Europäisches Patentamt

European Patent Office (11) Publication number: **0 072 880**

Office européen des brevets **B1**

(12) **EUROPEAN PATENT SPECIFICATION**

(45) Date of publication of patent specification: **16.10.85**

(21) Application number: **81303602.7**

(22) Date of filing: **06.08.81**

(51) Int. Cl.⁴: **A 61 K 45/06,** A 61 K 39/385 // (A61K39/385, 31:57)

(54) A composition for use in the control of ovulation of female bovines.

(43) Date of publication of application:
02.03.83 Bulletin 83/09

(45) Publication of the grant of the patent:
16.10.85 Bulletin 85/42

(84) Designated Contracting States:
AT BE CH IT LI LU NL SE

(56) References cited:
NATURE, vol. 269, 27 October 1977, London (GB), R.J. SCARAMUZZI et al.: "Increasing ovulation rate in sheep by active immunisation against an ovarian steriod androstenedione", pages 817-818
BIOLOGICAL ABSTRACTS, vol. 70, 1980, abstract no. 32667, Philadelphia, Pennsylvania (US), G.B. MARTIN et al.: "Effects of active immunization against androstenedione or estrone on estrus, ovulation and lambing in Merino ewes"
BIOLOGICAL ABSTRACTS, vol. 68, 1979, Philadelphia, Pennsylvania (US), R.W. ARMSTRONG et al.: "Effects of active immunization of female rabbits against testosterone", abstract no. 14896

(73) Proprietor: **COMMONWEALTH SCIENTIFIC AND INDUSTRIAL RESEARCH ORGANIZATION**
**Limestone Avenue**
**Campbell Australian Capital Territory 2601 (AU)**

(72) Inventor: **Cox, Ronald Ian**
**14 Park Avenue**
**Beecroft New South Wales (AU)**
Inventor: **Wilson, Patricia Ann**
**13 Landy Place**
**Beacon Hill New South Wales (AU)**
Inventor: **Hoskinson, Ronald Milton**
**Carcoola Crescent**
**Normanhurst New South Wales (AU)**
Inventor: **Mattner, Phillip Edward**
**1 Rose Street**
**Epping New South Wales (AU)**

(74) Representative: **Cockbain, Julian et al**
**Frank B. Dehn & Co. Imperial House 15-19,**
**Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# 0 072 880

**Description**

This invention relates to a process whereby the natural genetically determined ovulation rate of bovines may be manipulated and increased. In particular, it is concerned with immunization of bovines against androgenic and oestrogenic steroids in such a way that ovulation may be increased to a degree that significantly exceeds natural values. It is further concerned with the preparation and use of immunogenic compositions that can bring about the biological effects of the invention.

Because of their considerable economic importance, studies of the reproductive biology of domestic livestock have long been directed toward processes by which the individual fecundity of farm animals could be manipulated and in particular towards increasing the ovulation rate in females. The ovulation rate necessarily controls the maximum number of offspring that can be produced in a given pregnancy. Bovines have a very low natural ovulation rate, that is number of ovulations per animal occurring in the one oestrous cycle; for most breeds the ovulation rate lies between 1.00 and 1.01. Only in uncommon breeds are values such as 1.07 seen. Thus, bovines are very different in this aspect from many other domestic livestock species in which breeds often have ovulation rates of 2 and more. Clearly, for the natural fecundity of livestock to be increased, there must be at fertilization an increased ovulation rate.

It is already known in the prior art that, by artificially increasing the ovulation rate, in a domestic livestock species, by the administration of pregnant mare serum gonadotrophin (PMSG) for example, it is possible to increase the ovulation rate and ultimately the overall fecundity of the species. This process, however, suffers from the problem that the number of eggs shed cannot be controlled and the number of eggs shed by a cow treated with PMSG may vary from none up to 20.

Conjugates of testosterone with bovine serum albumin bonded by an oxime are already known (Biological Abstracts, Vol. 68, 14896 and Armstrong et al., J. Endocrinol 79(3), pp. 339—348 (1978)). These conjugates have proved to increase the number of ovulations in rabbits. Other similar conjugates such as oestrone or androstenedione-bovine serum albumin have already been promisingly tested in merino ewes. (Biological Abstract, Vol. 70, 32667 and Martin et al., Austr. J. Exp. Agric. Anim. Husb. 19(101), pp. 673—78, 1979 which confirmed the results of R. J. Scaramuzzi et al., Nature, 269, 27 October 1977, pp. 817—818). The conjugates used in the above experiments were prepared by conventional methods whereby the steroid, in the form of a derivative carrying an acidic grouping, is reacted with bovine serum albumin in the presence of a condensing agent and subsequently, the reaction mixture is extensively dialysed to remove all dialysable steroid material.

However, surprisingly, bovines have not responded to such immunizations with good antibody production nor with changes in ovulation rate when the same immunogens that promote ovulation rate increases in sheep and goats have been injected into the bovines.

We ourselves have now found that the presence of the steroid derivative involved in the conjugate in dialysable, loosely bonded form (i.e. not removed by dialysis as in prior preparations) dramatically enhances the efficiency of said conjugates in increasing the ovulation rate in bovines.

The present invention consists in a method for increasing the ovulation rate in female bovines comprising administering to the bovines an immunogenic composition for use in increasing the ovulation rate when administered to female bovines comprising (i) a conjugate of an acidic derivative of a steroid androgen or of an acidic derivative of a steroid oestrogen and an immunogenic protein, together with (ii) dialysable derivatives of said steroid androgen or oestrogen loosely bonded to said protein, the composition containing from 10 to 90% by weight of the steroid moiety, in the form of the dialysable loosely bonded steroid and (iii) an immunoadjuvant, so as to produce a mean steroid binding antibody titre in the female bovines of from 1 to 100 to 1 in 5,000 at the time of ovulation.

In another aspect the present invention consists in a composition efficacious in increasing the ovulation rate when administered to female bovines comprising (i) a conjugate of an acidic derivative of a steroid androgen or an acidic derivative of a steroid oestrogen and an immunogenic protein and (ii) dialysable derivatives of said steroid androgen or oestrogen loosely bonded to said protein, the composition containing from 10 to 90% by weight of the steroid moiety in the form of the dialysable loosely bonded steroid, and (iii) an immunoadjuvant.

As used in this specification, the following terms have meanings set out hereunder:

Steroid Androgen:
Any steroid substance that stimulates the expression of secondary sex characteristics of the male. Androgenic activity can be assessed by measuring the regrowth of the involuted comb of a castrated cock following androgen administration or by measuring the growth response of the seminal vesicles in castrated male rats following androgen administration.

Steroid Oestrogen:
Any steroid substance other than oestradiol- $17\beta$ that stimulates the expression of secondary sex characteristics in the female. Oestrogenic activity can be assessed by measurement of uterine growth or cornification of the vaginal epithelium following oestrogen administration to spayed female rats or mice. Immunisations against oestradiol- $17\beta$ have been found to render female animals anoestrus due to the

2

neutralisation of its powerful hormonal effects and it is therefore not suitable for use in the present invention.

Antibody titre: Defined here as the dilution of the antiserum which binds 50% of the maximum amount of labelled steroid bound by the antiserum or by the standard reference quality control antiserum during incubation of about 50 picograms of steroid for about 18 hours at 4°C followed by the use of either dextran-coated charcoal or polyethyleneglycol to separate free from antibody-bound steroid.

The immunizations of the invention are carried out with substances and procedures which are individually known in the art but which in combination are novel. Thus, to render the androgens or oestrogens immunogenic, suitable acidic derivatives are synthesized, for example derivatives of the steroids carrying an acidic group such as a hemisuccinyloxy, 2-carboxyethylthio or carboxymethoxy group, and these are linked by chemical means, covalently, to an immunogenic protein such as human serum albumin, ovalbumin, gelatin or γ-globulins typically, usually by means of a coupling agent e.g. a diimide such as 1-ethyl-3-(3-dimethylaminopropyl)carbodiimide.

To obtain effects on ovarian function and ovulation in bovines, it has been discovered, surprisingly, that the immunogens need to be prepared with particularly high levels of steroid incorporated into or associated with the carrier proteins. This is achieved by chemically reacting a large molar excess of the acidic steroid with the protein carrier. However, a high steroid incorporation alone is not a sufficient property of the immunogen to provide the effects of the invention. Commonly, the excess steroid used to facilitate a high molar steroid incorporation is deliberately removed from the conjugate by dialysis against aqueous, or aqueous organic, solvents or by column chromatography procedures. As indicated above, we have found, surprisingly, that the immunogenic compositions that achieve the effects of the invention are obtained if such dialysis is not pursued to completion to remove all the dialysable substances. It is part of its novelty that isolation of the immunogenic composition is achieved simply by incomplete dialysis of the reaction mixture against water alone for a period of 8—24 hours, optimally, followed by lyophilization.

Chemical analysis of the immunogenic composition so formed reveals that it is comprised of covalent steroid-protein conjugate together with dialysable steroid, loosely bound to protein, and a small proportion of unreacted steroid derivative. Surprisingly, the presence of the loosely bonded, dialysable steroid particularly enhances the immunogenic response to the steroid protein complex (Table 1). We have found that to produce an enhanced response, the immunogen composition should contain 10—90% and preferably 50—80% of the steroid moiety in loosely bonded, dialysable form. The compositions of this invention possess immunogenicity that is substantially greater than those skilled in the art would expect from a knowledge of the covalently linked hapten content.

The chemical procedure used to link the androgen or oestrogen to immunogenic protein can be any of the procedures known in the art which will at the same time provide an excess of dialysable steroid in addition to the covalently bound steroid. The immunogenic compositions so formed are administered to animals and the immune response to them potentiated with immuno-adjuvants such as DEAE-Dextran, the immunogen being in solution or suspension, or with the immunogen suspended rather than emulsified, in Freunds complete or incomplete adjuvant. Surprisingly, it has been found that emulsions of the immunogen in Freunds adjuvant are often poorly effective which may be because it is difficult to prepare an emulsion at the concentrations of immunogen required that is stable and remains stable for sufficiently long to produce the immunological responses after injection.

In preparation of the immunogenic compositions, substances already known in the art may be used. Thus, preferred androgen immunogens are prepared by the conjugation to protein of 4-androstene-3,17-dione derivatives functionalised with an acid group at positions 1, 7, 11 or 15 of the steroid ring, for example.

For immunization against testosterone, derivatives functionalized with an acid group at positions 3 or 17 may be used. The antibody titre range for androstenedione or testosterone that should be attained to achieve an increase in ovulation rate in bovines should preferably lie in the range 1:100 to 1:5000 as a mean for the herd. With antibody titres for these hormones which are significantly lower than this range, the incidence of multiple ovulations will tend to diminish and will approach that characteristic of unimmunized animals.

Suitable immunizations with the oestrogen, oestrone, can also promote an increase in the ovulation rate of bovines if titres lie in the range of 1 in 100 to 1 in 5,000 as a mean for the herd. The preferred steroid derivatives are those which, after conjugation with protein and administration to an animal, produce oestrone-specific antibody. Non-limiting examples of such oestrone derivatives already known in the art include oestrone-3-hemisuccinate, oestrone-3-carboxymethylether, oestrone-6-carboxymethyloxime and 15 carboxymethylestrone.

The invention places no limitation on the protein used to form the immunizing antigen of the invention but human serum albumin has been found effective.

The invention recognises that the range of antibody titres may vary with the hormone used in the immunization and with the breed of being immunized. The selection of the most preferred antibody titre within the above range is therefore a matter of experimentation in the particular circumstances of any particular application of this method.

The immunogenic composition is preferably administered by injections spaced apart by a period of from 1 to 5 weeks. In the case of an animal which has been previously immunised only a single injection

3

0 072 880

may be required. It is considered desirable to allow one full oestrus cycle to pass between the immunisation, or the second immunisation if that occurs, and mating of the animal. The immunogenic material should therefore be such that the desired antibody titre is obtained upon ovulation in an oestrus cycle separated from the time of immunisation by at least the length of one complete oestrus cycle.

In the examples given the biological effects achieved are a consequence of the development of antisteroid antibodies because immunizations of control animals against the protein carrier alone had no significant effect on the ovulation rate. In applying these effects to the breeding of bovines it will be recognised by those skilled in the art that they may conveniently be combined with synchronisation of the cycle, as with prostaglandins or progestagens, followed by artificial insemination, in which case there is no requirement for the bovines to show oestrus and recording of oestrus is not essential.

Further, they may conveniently be combined with both synchronization of oestrus and the use of luteinizing hormone releasing hormone, typically 66—90 h after the last synchronization treatment, followed by artificial insemination 6—12 h later again, in order to improve fertility through timing of ovulation as well as ensuring optimum ovulation rate.

The scope of this invention is not limited to processes employing active immunization protocols, for passive immunizations using antisteroid antibodies raised in donor animals may be used to achieve the increases in ovulation rate encompassed by the invention.

All the prior art immunization technologies that may be used to achieve the critical antisteroid antibody titres and the biological response of the invention are embraced by its method and processes.

The following example is given to illustrate preferred methods within the broad scope of this invention.

Example 1
(a) Preparation of steroid-protein immunogenic compositions

To an amount of 360 mg oestrone-3-carboxymethyl ether dissolved in 36 ml dioxane was added, dropwise and with stirring, a freshly prepared solution of 162 mg 1-ethyl-3-(3-dimethylaminopropyl) carbodiimide hydrochloride (ECDI) dissolved in 10.8 ml distilled water. The reaction was allowed to proceed for 30 minutes at 25°C and then a solution of 360 mg human serum albumin (HSA) dissolved in 48 ml phosphate buffer pH 7.80, 0.05 M was added dropwise with stirring. After 18 h a further solution of 180 mg oestrone-3-carboxymethylether in 12 ml dioxane was mixed with 54 mg ECDI in 3.6 ml water, allowed to stand 30 minutes at 25° and added to the main reaction mixture. The reaction was allowed to continue for $4\frac{1}{2}$ h and then a further 108 mg ECDI was added in solid form directly and with stirring to the mixture.

After a further 3 h at 25°C the reaction mixture was transferred to dialysis tubing and dialysed against distilled water. The water was changed after about 1, $1\frac{1}{2}$, 2 and 12 h. The product (oestrone immunogenic composition) retained in the dialysis sack was then lyophilized and weighed. Yield 400 mg. The steroid content of the steroid-protein immunogen was calculated by incorporating a trace amount of 3H-labelled oestrone-3-carboxymethylether in the reaction. By liquid scintillation counting of weighed amounts of the steroid derivative and the immunogen it was found that 120 moles steroid equivalent per mole protein were present in the immunogenic composition; 35 moles steroid/mole protein were covalently linked, the remainder being predominantly dialysable steroid loosely bound to protein.

In a similar preparation 7α-(2-carboxyethylthio)-4-androstene-3,17-dione was linked to human serum albumin and yielded 380 mg of product (androstenedione immunogen composition) having 90 moles steroid equivalent per mole protein present of which 32 moles were covalently linked and the remainder being predominantly dialysable steroid loosely bound to the protein.

In a similar preparation testosterone-3-carboxymethyloxime was linked to human serum albumin and yielded 385 mg of product (testosterone immunogenic composition) having 102 moles steroid equivalent per mole of protein present, of which 33 moles were covalently linked, the remainder being predominantly dialysable steroid loosely bound to the protein.

(b) Immunization of bovines to test steroid immunogenic composition

100—250 mg of oestrone, androstenedione or testosterone immunogenic compositions were pasted in a 1 ml 0.9% sterile saline and made into a total volume of 15 ml 0.9% sterile saline. Then 15 ml DEAE-dextran solution was added. The DEAE dextran solution was prepared by dissolving 15 g in 100 ml water and adjusting the pH to 7.5—7.7 using saturated tri-(hydroxy-methyl)-methylamine buffer (500 g/litre water). The final volume of the DEAE dextran solution was then adjusted to 150 ml.

Alternatively, 100—250 mg of steroid immunogenic compositions were pasted in 1 ml Freunds adjuvant and made into a suspension in a total volume of 30 ml in Freunds adjuvant.

Bovines (of Hereford and Hereford-Friesan breeds) were injected each with 3 ml of the vaccine, containing 10 or 25 mg immunogen, each animal being given injections at about 10 sites subcutaneously over the neck region. An intramuscular injection of 1.5 ml pertussis vaccine was also administered. The injection treatment was repeated 4 weeks later.

Blood samples were taken by tail vein puncture one week after the second treatment. Blood was collected into heparinized tubes, stored on ice and centrifuged at 4°C. The plasma so obtained was stored at −10°C until analysis of steroid antibody titre. The steroid-antibody titres obtained from the immunogenic compositions in (a) are shown in Table 1, together with the low values obtained with conventionally prepared steroid-protein conjugates.

4

**0 072 880**

Example 2

Immunization of bovines against steroids and effect on ovarian size and activity

Using immunogenic compositions and methods as described in Example 1 bovines were treated and examined. Ovarian changes were assessed by palpation per rectum in the period 8—17 weeks after commencing treatment and the number and size of follicles or corpora lutea on left and right ovaries were noted. Occurrence of oestrus in the bovines was determined using K-Mar heat mount detectors; bovines generally continued to show oestrus following treatment.

The results of immunization are given in Table 2, showing a marked increase in ovarian activity, follicle number and numbers of corpora lutea with immunization against oestrone and androstenedione compared to control animals.

TABLE 1

Enhanced immunogenicity of compositions containing dialysable steroid in addition to steroid protein conjugates

| Steroid derivative used in making immunogen composition | Total steroid moiety present moles/mole protein | Covalently linked hapten content | Number of of animals treated | Mean steroid antibody titre produced[*] for group |
|---|---|---|---|---|
| Testosterone-3-carboxymethyl oxime | 33# 102+ | 33 33 | 5 8 | Below 1:50 1:865 |
| Androstenedione-7α-carboxyethyl thioether | 33# 90+ | 33 32 | 6 6 | Below 1:50 1:300 |
| Oestrone-3-carboxymethylester | 120+ | 35 | 6 | 1:6420 |

[*] Following 2 treatments—primary and secondary
+ These preparations contain a high proportion of dialysable steroid
# Preparations of steroid-protein conjugate conventionally dialysed to completely remove excess steroid.

TABLE 2

Ovarian changes in cattle 11 weeks after start of treatment

| Steroid derivative used in making immunogenic composition | Number of animals | Animals with enlarged ovaries | Animals with multiple corpora lutea or several large follicles on the ovaries |
|---|---|---|---|
| Oestrone-3-carboxymethylether | 9 | 5 | 5 |
| Androstenedione-7α-carboxy-ethylthioether | 6 | 4 | 2 |
| Controls (Untreated) | 10 | 1 | 0 |

Example 3

Using immunogenic compositions and methods as described in Example 1 to maintain titres but including repeated treatments a further series of tests with immunizations against testosterone, androstenedione or oestrone was carried out. Palpation per rectum indicated ovarian responses and these were then confirmed and detailed by endoscopic examination of the bovines (Table 3) in which the ovaries were visualized and size, numbers of follicles and corpora lutea were recorded.

5

TABLE 3
Endoscopic examination of bovines following immunization against steroids
and showing resultant increases in ovulation rate

| Immunization against (steroid | Number of animals in groups | Mean steroid antibody titre | Number of animals having 2 or 3 ovulations | |
|---|---|---|---|---|
| Testosterone | 7 | 270 | 3 | |
| Androstenedione | 6 | 300 | 2 | |
| Oestrone | 8 | 1500 | 1 | |
| Total of Immunized Animals | 21 | — | 6 (29%) | |
| Control (Reference) | 2 | 0 | 0 | 0 |

Only a small control group was run in this series as it is well established that the multiple ovulation rate in untreated bovines of the Hereford or Hereford-Friesian breeds is very low and about 1% i.e. the untreated bovines have an ovulation rate of 1.01 as compared with the ovulation rate of the total of immunized animals in this example of 1.29.

**Claims**

1. An immunogenic composition for use in increasing the ovulation rate when administered to female bovines comprising (i) a conjugate of an acidic derivative of a steroid androgen or of an acidic derivative of a steroid oestrogen and an immunogenic protein, together with (ii) dialysable derivatives of said steroid androgen or oestrogen loosely bonded to said protein, the composition containing from 10 to 90% by weight of the steroid moiety in the form of the dialysable loosely bonded steroid, and (iii) an immunoadjuvant.

2. A composition according to claim 1 wherein the immunogenic composition contains at least 50% by weight of the steroid moiety in the form of said dialysable loosely bonded steroid.

3. A composition according to claim 1 or claim 2 wherein the immunogenic composition contains up to 80% by weight of the steroid moiety in the form of said dialysable loosely bonded steroid.

4. A composition according to any one of claims 1 to 3 wherein the steroid androgen or oestrogen is selected from 4-androstene-3,17-dione, testosterone and oestrone.

5. A composition according to any one of the preceding claims wherein the immunogenic protein is selected from human serum albumin, ovalbumin, gelatin and γ-globulin.

6. A composition according to any one of the preceding claims in which the conjugate contains 25 to 35 moles of acidic steroid androgen or oestrogen derivative per mole of immunogenic protein.

7. A composition according to any one of claims 1 to 6 wherein the acidic steroid androgen or oestrogen derivative is selected from 4-androstene-3,17-dione-7α-(2-carboxyethylthioether), testosterone-3-carboxymethyloxime and oestrone-3-carboxymethylether.

8. A composition according to any one of the preceding claims wherein the immuno-adjuvant is selected from diethylaminoethyldextran, Freunds complete adjuvant and Freunds incomplete adjuvant.

9. A composition according to claim 8 wherein the steroid androgen derivative is 4-androstene-3,17-dione-7α-(2-carboxyethylthioether) and the immunoadjuvant is diethylaminoethyldextran.

10. A composition according to any one of the preceding claims wherein the immuno-adjuvant is selected from diethylaminoethyldextran, Freunds complete adjuvant and Freunds incomplete adjuvant and the immunogenic protein is human serum albumin.

11. Use of a composition according to any one of the preceding claims for increasing the ovulation rate in female bovines.

12. Use of a composition according to any one of claims 1 to 10 for increasing the ovulation rate in female bovines in which oestrus is synchronised by administration of a prostaglandin or prostagen and in which the bovines are artificially inseminated from 72 to 96 hours after the administration of such prostaglandin or prostagen.

13. The use of a composition according to claim 11 in which oestrus in the bovines is synchronised by administration of a prostaglandin or prostagen, and in which luteinising hormone releasing hormone is administered to the bovines from 66 to 90 hours after the administration of the oestrus-synchronising prostaglandin or prostagen and the bovines are artificially inseminated from 6 to 12 hours after administration of the luteinising hormone releasing hormone.

**Patentansprüche**

1. Immunogene Zusammensetzung zur Verwendung bei der Erhöhung der Ovulationsrate, wenn man sie weiblichen Rindern verabreicht, gekennzeichnet durch einen Gehalt an (i) einem Konjugat eines sauren Derivates eines Steroidandrogens oder eines sauren Derivates eines Steroidöstrogens und einem immunogenen Protein, zusammen mit (ii) dialysierbaren Derivaten des genannten Steroidandrogens oder Östrogens, lose gebunden an das genannte Protein, wobei die Zusammensetzung von 10 bis 90 Gew.-% des Steroidteiles in Form des dialysierbaren, lose gebundenen Steroids enthält, und (iii) einem Immunohilfsmittel.

2. Zusammensetzung nach Anspruch 1, dadurch gekennzeichnet, daß die immunogene Zusammensetzung mindestens 50 Gew.-% des Steroidteiles in Form des genannten dialysierbaren, lose gebundenen Steroids enthält.

3. Zusammensetzung nach Anspruch 1 oder 2, dadurch gekennzeichnet, daß die immunogene Zusammensetzung bis zu 80 Gew.-% des Steroidteiles in Form des genannten dialysierbaren, lose gebundenen Steroids enthält.

4. Zusammensetzung nach einem der Ansprüche 1 bis 3, dadurch gekennzeichnet, daß das Steroidandrogen oder Östrogen unter 4-Androsten-3,17-dion, Testosteron und Östron ausgewählt ist.

5. Zusammensetzung nach einem der vorhergehenden Ansprüche, dadurch gekennzeichnet, daß das immunogene Protein unter menschlichem Serumalbumin, Ovalbumin, Gelatine und γ-Globulin ausgewählt ist.

6. Zusammensetzung nach einem der vorhergehenden Ansprüche, dadurch gekennzeichnet, daß das Konjugat 25—35 Mole des sauren Steroidandrogen- oder Östrogenderivates pro Mol des immunogenen Proteins enthält.

7. Zusammensetzung nach einem der Ansprüche 1 bis 6, dadurch gekennzeichnet, daß das saure Steroidandrogen- oder Östrogenderivat unter 4-Androsten-3,17-dion-7α-(2-carboxyethylthioether), Testosteron-3-carboxymethyloxim und Östron-3-carboxymethylether ausgewählt ist.

8. Zusammensetzung nach einem der vorhergehenden Ansprüche, dadurch gekennzeichnet, daß das Immunohilfsmittel unter Diethylaminoethyldextran, Freunds vollständigem Hilfsmittel und Freunds unvollständigem Hilfsmittel ausgewählt ist.

9. Zusammensetzung nach Anspruch 8, dadurch gekennzeichnet, daß das Steroidandrogenderivat 4-Androsten-3,17-dion-7α-(2-carboxyethylthioether) und das Immunohilfsmittel Diethylaminoethyldextran ist.

10. Zusammensetzung nach einem der vorhergehenden Ansprüche, dadurch gekennzeichnet, daß das Immunohilfsmittel unter Diethylaminoethyldextran, Freunds vollständigem Hilfsmittel und Freunds unvollständigem Hilfsmittel ausgewählt ist und daß das immunogene Protein menschliches Serumalbumin ist.

11. Verwendung einer Zusammensetzung nach einem der vorhergehenden Ansprüche zur Erhöhung der Ovulationsrate bei weiblichen Rindern.

12. Verwendung einer Zusammensetzung nach einem der Ansprüche 1—10 zur Erhöhung der Ovulationsrate bei weiblichen Rindern, wobei der Oestrus durch Verabreichung eines Prostaglandins oder Prostagens synchronisiert wird und wobei die Rinder 72—96 Stunden nach der Verabreichung eines solchen Prostaglandins oder Prostagens künstlich besamt werden.

13. Verwendung einer Zusammensetzung nach Anspruch 11, wobei der Oestrus der Rinder durch Verabreichung eines Prostaglandins oder Prostagens synchronisiert wird und wobei das das luteinisierende Hormon freisetzende Hormon den Rindern 66—90 Stunden nach der Verabreichung des Oestrus synchronisierenden Prostaglandins oder Prostagens verabreicht wird und die Rinder 6—12 Stunden nach Verabreichung des das luteinisierende Hormon freisetzenden Hormons künstlich besamt werden.

**Revendications**

1. Composition immunogène pour augmenter le taux d'ovulation lorsqu'on l'administre à des bovidés femelles, caractérisée en ce qu'elle comprend (i) un conjugué d'un dérivé acide d'un androgène stéroïde ou d'un dérivé acide d'un oestrogène stéroïde et d'une protéine immunogène, en même temps que (ii) des dérivés dialysables de l'oestrogène et de l'androgène stéroïdes précités liés lâchement à la protéine susmentionnée, la composition contenant de 10 à 90% en poids de la fraction stéroïde sous la forme du stéroïde lâchement lié dialysable et (iii) un immunoadjuvant.

2. Composition suivant la revendication 1, caractérisée en ce qu'elle contient au moins 50% en poids de la fraction stéroïde sous la forme du stéroïde lâchement lié dialysable précité.

3. Composition suivant l'une quelconque des revendications 1 et 2, caractérisée en ce qu'elle contient jusqu'à 80% en poids de la fraction stéroïde sous la forme du stéroïde lâchement lié dialysable précité.

4. Composition suivant l'une quelconque des revendications 1 à 3, caractérisée en ce que l'oestrogène et l'androgène stéroïdes sont choisis parmi la 4-androstène-3,17-dione, la testostérone et l'oestrone.

5. Composition suivant l'une quelconque des revendications précédentes, caractérisée en ce que l'on choisit la protéine immunogène parmi la γ-globuline, la gélatine, l'ovalbumine et l'albumine sérique humaine.

6. Composition suivant l'une quelconque des revendications précédentes, caractérisée en ce que le conjugué contient de 25 à 35 moles de dérivé d'oestrogène ou d'androgène stéroïde acide par mole de protéine immunogène.

7. Composition suivant l'une quelconque des revendications 1 à 6, caractérisée en ce que le dérivé d'oestrogène ou d'androgène stéroïde est choisi parmi le 4-androstène-3,17-dione-7α-(2-carboxyéthylthioéther), la testostérone-3-carboxyméthyloxime et l'oestrone-3-carboxyméthyléther.

8. Composition suivant l'une quelconque des revendications précédentes, caractérisée en ce que l'immunoadjuvant est choisi parmi le diéthylaminoéthyldextrane, l'adjuvant complet de Freund et l'adjuvant incomplet de Freund.

9. Composition suivant la revendication 8, caractérisée en ce que le dérivé androgène stéroïde est le 4-androstène-3,17-dione-7α-(2-carboxyéthylthioéther) et l'immunoadjuvant est le diéthylaminoéthyldextrane.

10. Composition suivant l'une quelconque des revendications précédentes, caractérisée en ce que l'immunoadjuvant est choisi parmi le diéthylaminoéthyldextrane, l'adjuvant complet de Freund et l'adjuvant incomplet de Freund et la protéine immunogène est l'albumine sérique humaine.

11. Utilisation d'une composition suivant l'une quelconque des revendications précédentes pour augmenter le taux d'ovulation des bovidés femelles.

12. Utilisation d'une composition suivant l'une quelconque des revendications 1 à 10 pour augmenter le taux d'ovulation des bovidés femelles, caractérisé en ce que le rut ou oestrus est synchronisé par l'administration d'une prostaglandine ou d'un prostagène et en ce que les bovidés subissent une insémination artificielle de 72 à 96 heures après l'administration de ladite prostaglandine ou dudit prostagène.

13. Utilisation d'une composition suivant la revendication 11, caractérisée en ce que l'oestrus des bovidés est synchronisé par l'administration d'une prostaglandine ou d'un prostagène et en ce que l'hormone libératrice de l'hormone lutéinisante est administrée aux bovidés de 66 à 90 heures après l'administration de la prostaglandine ou du prostagène synchronisant le rut ou l'oestrus et les bovidés subissent une insémination artificielle de 6 à 12 heures après l'administration de l'hormone libératrice de l'hormone lutéinisante.